Europäisches Patentamt

European Patent Office

Office européen des brevets

(11) Veröffentlichungsnummer: **0 407 634 B1**

## EUROPÄISCHE PATENTSCHRIFT

(45) Veröffentlichungstag der Patentschrift: **28.07.93**

(51) Int. Cl.⁵: **A61K 35/78**

(21) Anmeldenummer: **89112639.3**

(22) Anmeldetag: **11.07.89**

---

(54) **Schmerzstillendes und entzündungshemmendes Arzneimittel.**

---

(43) Veröffentlichungstag der Anmeldung:
**16.01.91 Patentblatt 91/03**

(45) Bekanntmachung des Hinweises auf die
Patenterteilung:
**28.07.93 Patentblatt 93/30**

(84) Benannte Vertragsstaaten:
**AT BE CH DE ES FR GB GR IT LI LU NL SE**

(56) Entgegenhaltungen:

**ARZNEIMITTELFORSCHUNG, Band 39, Nr. 6, Juni 1989, Seiten 689-703; S.N. OKPANYI et al.: "Antiphlogistische, analgetische und antipyretische Wirkung unterschiedlicher Pflanzenextrakte und deren Kombination im Tiermodell"**

(73) Patentinhaber: **STEIGERWALD ARZNEIMITTEL-WERK GMBH**
**Havel Strasse 5**
**W-6100 Darmstadt(DE)**

(72) Erfinder: **Krauskopf, Jobst**
**Lessingstrasse 19**
**W-6146 Bensheim 3(DE)**

(74) Vertreter: **Rupprecht, Klaus, Dipl.-Ing.**
**Kastanienstrasse 18**
**W-6242 Kronberg (DE)**

**Beschreibung**

Die Erfindung bezieht sich auf ein schmerzstillendes und entzündungshemmendes Arzneimittel.

In der Therapie der entzündlichen rheumatologischen Erkrankungen, insbesondere der rheumatoiden Arthritis, werden zwei verschiedene medikamentöse Prinzipien nebeneinander verwendet. Das erste besteht in der Verminderung des Schmerzes und der entzündlichen Erscheinungen am betroffenen Gelenk. Dazu dienen verschiedene Substanzklassen, z. B. Salizylsäurederivate, Essig- und Propionsäurederivate oder Oxicame. Eine zusammenfassende Bezeichnung ist "nichtsteroidale Antirheumatika" (NSAR). Ihre Wirksamkeit wird am betroffenen Gelenk selbst beurteilt. Die Effekte treten rasch (Stunden bis Tage) auf. Die Dosis wird der augenblicklichen Situation angepaßt. Eine Verlaufsänderung der entzündlichen rheumatischen Erkrankung tritt durch diese Medikamente nicht ein.

Das in dem WIPO-Dokument (WO 87/022 48) beschriebene Arzneimittel auf rein pflanzlicher Basis besitzt bei besserer Verträglichkeit die Wirkungen der nichtsteroidalen Antirheumatika (NSAR) ebenfalls.

Neben der beschriebenen Wirksamkeit sind Mittel bekannt und werden intensiv weiter gesucht, die den Gesamtverlauf der chronisch-unheilbaren Erkrankungen günstig beeinflussen (sog. disease modifying drugs). Sie beeinflussen Schmerzen und Entzündungen erst nach (monate-) langer Verabreichung unter Erhaltungsdosen und sind mit erheblichen Nebenwirkungen behaftet (etwa ein Drittel der Patienten muß diese Behandlung abbrechen). Ihre Wirksamkeit wird zunächst an einer Verbesserung der systemischen Entzündungszeichen erkannt. Dies ist insbesondere eine Verbesserung der Blutkörperchen-Senkungs-Reaktion, die allgemein als Methode zur Einschätzung eines Entzündungsgeschehens verwendet wird. Andere Reaktionen, die keine prinzipiell anderen Aussagen ergeben, sind z. B. das C-reaktive Protein (CPR). Langfristiges Ziel bei dieser Behandlung ist die Verminderung oder Vermeidung der Gelenkzerstörung durch die rheumatische Entzündung. Diese Therapie ist unter dem Namen Basistherapie heute geläufig.

Wegen häufiger und gelegentlich kritischer Nebenwirkungen gibt es für die Behandlung mit Basistherapeutika sorgsam ausgearbeitete Regeln. Beispielhaft sollen für die Änderung der Blutkörperchen-Senkungs-Reaktion Ergebnisse aus einer 20-Wochen-Doppelblindstudie angeführt werden (Ward, J. R. et al., Arthritis and Rheumatism Vol. 26, No. 11, 1983, Seite 1303 - 1315).

| Medikament | BKS vor Therapie | BKS nach 20 Wochen |
|---|---|---|
| Placebo | 55 | 51 |
| Auranofin | 63 | 22 |
| Gold-Na-Thiomalate | 58 | 33 |
| BKS = Blutkörperchensenkungsgeschwindigkeit in min/h nur für die erste Stunde | | |

Überraschenderweise wurde nunmehr bei einer Untersuchung in verschiedenen Forschungszentren gefunden, daß basistherapeutische Wirkungen auch mit dem im WO 87/02248 geoffenbarten Arzneimittel erzielt wurden.

Dieses Arzneimittel mit schmerzstillender und entzündungshemmender Wirkung auf pflanzlicher Basis zeichnet sich aus durch die Bestandteil Populus tremula, Solidago virgaurea, Fraxinus excelsior als alleine Träger der Wirksamkeit.

Als vorteilhaft hat es sich erwiesen, wenn das erfindungsgemäße Arzneimittel 50 - 70 Vol.-% Populus tremula, 10 - 30 Vol.-% Solidago virgaurea, 10 - 30 Vol.-% Fraxinus excelsior aufweist.

Eine besonders bevorzugte Zusammensetzung des erfindungsgemäßen Arzneimittels weist 60 Vol.-% Populus tremula, 20 Vol.-% Solidago virgaurea, 20 Vol.-% Fraxinus excelsior auf.

Schließlich liegt es im Rahmen der Erfindung, daß in dem Bestandteil Populus tremula Rinde und Blätter im Verhältnis 1 : 2 vorhanden sind.

Die vorliegende Erfindung besteht in der überraschenden Erkenntnis, daß dieses Arzneimittel nicht nur zur unmittelbaren Behandlung von rheumatischen Erkrankungen eingesetzt werden kann, sondern vielmehr insbesondere auch als Basistherapeutikum.

Als Beispiel zur erfindungsgemäß erkannten basistherapeutischen Wirkung des Arzneimittels werden Ergebnisse von Untersuchungen in der rheumatologischen Abteilung eines Krankenhauses wiedergegeben, und zwar anhand der CRP-Werte des Arzneimittels nach WO 87/02248. Es zeigt sich eine deutliche Senkung dieser Werte. Mit Sicherheit kann gesagt werden, daß sich aufgrund der Gaben des Arzneimittels WO 87/02248 die entzündliche Aktivität der rheumatologischen Erkrankungen - ausgedrückt durch das C-reaktive Protein - deutlich verminderte. Nachstehend einige Ergebnisse tabellarisch:

CRP-Werte

| Zeitraum | Mittelwert | ± | Standardabweichung |
|---|---|---|---|
| vor WO 87/02248 | 46,2 | | 32,1 |
| nach 3 Monaten | 37,4 | | 30,9 |
| nach 6 Monaten | 32,9 | | 26,4 |
| nach 9 Monaten | 29,4 | | 21,8 |
| nach 12 Monaten | 27,9 | | 21,3 |

Es muß dabei bedacht werden, daß die hier untersuchten Patienten in beiden Gruppen zusätzlich eine bei Beginn der Prüfung schon lange durchgeführte Langzeitbehandlung mit "Basistherapie" erhielten. Diese wurde nicht verändert, da die Entdeckung nicht vorhersehbar war. Die Effekte durch das Arzneimittel auf pflanzlicher Basis nach WO 87/02248 gehen folglich über bereits erreichte Wirkungen hinaus. Dabei geht die Gruppe des Arzneimittels auf pflanzlicher Basis nach WO 87/02248 von ungünstigeren Werten aus als die Placebogruppe.

Unter diesen Gegebenheiten ist eine Senkung der Blutkörperchen-Senkungs-Reaktionvon 31 % ein beachtlicher Effekt.

Bei einer Überprüfung an 5 bereits behandelten Patienten mit rheumatoider Arthritis ergab sich, daß durch Hinzu-Verabreichung des Arzneimittels auf pflanzlicher Basis nach WO 87/02248 die Ausgangswerte in drei von fünf Fällen bei der Blutkörperchen-Senkungs-Reaktion und in vier von fünf Fällen beim C-reaktiven Protein innerhalb von vier Wochen Veränderungen nach unten, d.h. in therapeutischer Richtung, eintreten. Dieser deutliche Effekt überrascht aus mehreren Gründen:

1. Die hier verwendeten Dosierungen sind nicht im Hinblick auf basistherapeutische Wirksamkeit ermittelt worden. Sie entsprechen vielmehr des Dosis bei symptomatischer Therapie.

2. Die beobachteten Wirkungen sind zusätzliche Wirkungen bei bereits durchgeführter Basistherapie. Bei der Entdeckung wäre es ethisch nicht vertretbar gewesen, Schwerkranken eine bekannt wirksame Therapie vorzuenthalten. Es war gänzlich unerwartet, daß sich über diese traditionelle Therapie hinaus eine weitere Verstärkung der basistherapeutischen Wirksamkeit ergeben könnte.

3. Bei der Würdigung der Entdeckung muß bedacht werden, daß das Arzneimittel auf pflanzlicher Basis nach WO 87/02248 gegenüber anderen Antirheumatika wesentlich besser verträglich ist. Daraus ergibt sich die Möglichkeit.

3.1 sowohl über höhere Dosen zu stärkeren Effekten zu kommen als auch

3.2 durch Hinzuverwendung des Arzneimittels nach WO 87/02248 neben traditioneller Basistherapie deren Effektivität weiter zu erhöhen.

**Patentansprüche**

1. Verwendung eines Stoffgemisches auf pflanzlicher Basis mit den Bestandteilen Populus tremula, Solidago virgaurea, Fraxinus excelsior als alleinige Träger der Wirksamkeit zur Herstellung eines Arzneimittels für eine basistherapeutische Anwendung bei rheumatologischen Erkrankungen.

2. Verwendung des Stoffgemisches zur Herstellung des Arzneimittels nach Anspruch 1, mit
   50 - 70 Vol.-% Populus tremula
   10 - 30 Vol.-% Solidago virgaurea
   10 - 30 Vol.-% Fraxinus excelsior.

3. Verwendung des Stoffgemisches zur Herstellung des Arzneimittels nach Anspruch 1 oder 2, mit
   60 Vol.-% Populus tremula
   20 Vol.-% Solidago virgaurea
   20 Vol.-% Fraxinus excelsior.

4. Verwendung des Stoffgemisches zur Herstellung des Arzneimittels nach einem der Ansprüche 1 bis 3, wobei in dem Bestandteil Populus tremula Rinde und Blätter im Verhältnis 1 : 2 vorhanden sind.

**Claims**

1.   Use of a mixture of substances on a plant basis with the constituents
     Populus tremula
     Solidago virgaurea
     Fraxinus excelsior as the sole carrier of the activity for the production of a medicament for a basic therapeutic application for rheumatological diseases.

2.   Use of the mixture of substances for the production of the medicament according to Claim 1, with
     50 - 70 vol.-% of Populus tremula
     10 - 30 vol.-% of Solidago virgaurea
     10 - 30 vol.-% of Fraxinus excelsior.

3.   Use of the mixture of substances for the production of the medicament according to Claim 1 or 2, with
     60 vol.-% of Populus tremula
     20 vol.-% of Solidago virgaurea
     20 vol.-% of Fraxinus excelsior.

4.   Use of the mixture of substances for the production of the medicament according to one of Claims 1 to 3, in which bark and leaves are present in the rato 1 : 2 in the constituent Populus tremula.

**Revendications**

1.   Application d'un mélange de produits à base de plantes avec les composants suivants :
     populus tremula,
     solidago virgaurea,
     fraxinus excelsior, comme seules substances actives pour la fabrication d'un médicament destiné à des applications à base thérapeutique pour des maladies rhumatismales.

2.   Utilisation du mélange de produits pour la fabrication du médicament selon la revendication 1, avec :
     50 - 70 % en volume de populus tremula,
     10 - 30 % en volume de solidago virgaurea,
     10 à 30 % en volume de fraxinus excelsior.

3.   Application du mélange de produits pour la fabrication d'un médicament selon les revendications 1 ou 2, avec :
     60 % en volume de populus tremula,
     20 % en volume de solidago virgaurea,
     20 % en volume de fraxinus excelsior.

4.   Application du mélange de produits pour la fabrication du médicament selon les revendications 1 à 3, le composant populus tremula étant de l'écorce et des feuilles, pris dans un rapport de 1 : 2.